# EUROPÄISCHE PATENTSCHRIFT

(11) **EP 1 787 684 B1**
(45) Veröffentlichungstag und Bekanntmachung des Hinweises auf die Patenterteilung: **20.01.2016**
(21) Anmeldenummer: 06023409.3
(22) Anmeldetag: 10.11.2006
(51) Int. Cl.: A61Q 5/10, A61K 8/41, C07C 271/28, C07C 215/16, C07D 413/10

(54) **Neue Kupplerkomponenten**
New couplers
Nouveaux coupleurs

(30) Priorität: 22.11.2005 DE 102005055892
(43) Veröffentlichungstag der Anmeldung: 23.05.2007
(73) Patentinhaber: Henkel AG & Co. KGaA, 40589 Düsseldorf (DE)
(72) Erfinder: Knübel, Georg, 40219 Düsseldorf (DE); Nemitz, Ralph, 41363 Jüchen (DE); Höffkes, Horst, 40595 Düsseldorf (DE)

(56) Entgegenhaltungen:
- WO-A-2005/035499
- WO-A-2005/042505
- DE-A1- 10 103 160
- GB-A- 2 148 921
- JP-A- 62 043 465

## Beschreibung

Die vorliegende Erfindung betrifft Mittel zur Färbung keratinischer Fasern, die spezielle m-Phenylendiaminderivate enthalten, die in 5-Stellung ein Halogenatom tragen, ein Verfahren zur Färbung von Haaren mit diesen Mitteln, sowie eines dieser m-Phenylendiaminderivate selbst und Zwischenprodukte, die bei der Herstellung dieser Verbindung entstehen.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Die Färbung keratinischer Fasern unter Einsatz spezifischer m-Phenylendiamine beschreibt beispielsweise die deutsche Patentanmeldung DE 101 03160 A1 (Henkel).

Halogenierte m-Phenylendiaminderivate können nach Lehre der japanischen Patentanmeldung JP 62 043465 (Mitsubishi Chem Ind) und der britischen Patentanmeldung GB 2 148 921 (Nippon Kayaku KK) zur Färbung von Cellulosefasern eingesetzt werden.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluss chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, die auch in toxikologischer und dermatologischer Hinsicht unproblematisch sind.

Aufgabe der vorliegenden Erfindung war es daher, neue Kupplerkomponenten zu entwickeln, die die an Oxidationsfarbstoffvorprodukte gestellten Anforderungen, insbesondere hinsichtlich der toxikologischen und dermatologischen Eigenschaften, erfüllen und Färbungen in einem breiten Farbspektrum mit guten Echtheitseigenschaften ermöglichen.

Überraschenderweise wurde nun gefunden, dass spezielle m-Phenylendiaminderivate, die in 5-Stellung ein Halogenatom tragen, den an Kupplerkomponenten gestellten Anforderungen in einem hohen Maße genügen. Die erfindungsgemäßen Kupplerkomponenten ermöglichen insbesondere mit den Entwicklerkomponenten p-Toluylendiamin, 1-(2-Hydroxyethyl)-2,5-diaminobenzol, Bis-(2-hydroxy-5-aminophenyl)-methan, 2,4,5,6-Tetraaminopyrimidin und N,N-Bis-(2-hydroxyethyl)-p-phenylendiamin intensive Färbungen mit guten Echtheitseigenschaften.

Ein erster Gegenstand der vorliegenden Erfindung sind daher Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 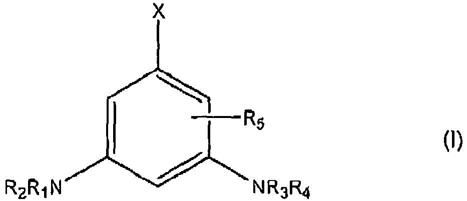 wobei
R₁, R₂, R₃ und R₄ unabhängig voneinander für ein Wasserstoffatom, eine verzweigte oder unverzweigte C₁- bis C₄-Alkylgruppe, eine verzweigte oder unverzweigte C₁- bis C₄-Monohydroxyalkylgruppe, eine verzweigte oder unverzweigte C₂- bis C₄- Polyhydroxyalkylgruppe, eine verzweigte oder unverzweigte C₁- bis C₄-Alkoxy-(C₁- bis C₄)-alkylgruppe, eine verzweigte oder unverzweigte C₁- bis C₄-Aminoalkylgruppe, eine verzweigte oder unverzweigte C₁- bis C₄-Alkykamino-(C₁- bis C₄)-alkylgruppe oder eine verzweigte oder unverzweigte C₁- bis C₄-Dialkykamino-(C₁- bis C₄)-alkylgruppe stehen,
R₅ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe, eine C₁- bis C₄-Alkoxy-(C₁- bis C₄)-alkylgruppe oder ein Halogenatom, und
X steht für ein Halogenatom,
mit den Maßgaben, dass
- mindestens einer der Substituenten R₁, R₂, R₃ und R₄ für Wasserstoff steht, und
- mindestens einer der Substituenten R₁, R₂, R₃ und R₄ von Wasserstoff verschieden ist.

Unter keratinischen Fasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Da es sich bei den erfindungsgemäßen m-Phenylendiaminderivaten um AminoVerbindungen handelt, lassen sich aus diesen in üblicher Weise die bekannten Säureadditionssalze herstellen. Alle Aussagen dieser Schrift und demgemäss der beanspruchte Schutzbereich beziehen sich daher sowohl auf die in freier Form vorliegenden Verbindungen als auch auf deren wasserlösliche, physiologisch verträglichen Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Beispiele für die als Substituenten in den erfindungsgemäßen Verbindungen genannten C₁- bis C₆-Alkylgruppen sind die Gruppen Methyl, Ethyl, Propyl, Isopropyl, n-Butyl, 2-Methylpropyl, Ethyl und Methyl sind bevorzugte Alkylgruppen. Bevorzugte C₁- bis C₄-Alkoxygruppen sind die Gruppen Methoxy und Ethoxy. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die übrigen verwendeten Bezeichnungen leiten sich von den hier gegebenen Definitionen ab.

Die m-Phenylendiaminderivate der Formel (I) lassen sich mit Hilfe herkömmlicher organischer Methoden herstellen. Beispielhaft sei an dieser Stelle auf die Versuchsdurchführungen im Rahmen der Ausführungsbeispiele verwiesen.

In den deutschen Offenlegungsschriften DE 198 28 204 und DE 198 21 535 wird der Grundkörper 5-Chlor-m-phenylendiamin als Kupplerkomponente in oxidativen Färbesystemen beschrieben. Dieser Schrift sind aber keinerlei Hinweise darauf zu entnehmen, dass die Derivate dieses Grundkörpers, die an mindestens einem Stickstoffatom eine Alkylgruppe tragen, sich durch die nunmehr gefundenen hervorragenden färberischen Eigenschaften auszeichnen. Während die unsubstituierten Vertreter (beispielsweise 1-Chlor-3,5-diaminobenzol in Kombination mit p-Toluylendiamin) zu Färbungen im Braunbereich führen, ermöglichen die erfindungsgemäßen Kupplerkomponenten intensive und brillante Rottöne.

Bei den erfindungsgemäßen m-Phenylendiaminderivaten der Formel (I) stehen die Substituenten R₁, R₂, R₃ und R₄ unabhängig voneinander für ein Wasserstoffatom, eine verzweigte oder unverzweigte C₁- bis C₄-Alkylgruppe, eine verzweigte oder unverzweigte C₁- bis C₄-Monohydroxyalkylgruppe, eine verzweigte oder unverzweigte C₂- bis C₄- Polyhydroxyalkylgruppe, eine verzweigte oder unverzweigte C₁-bis C₄-Alkoxy-(C₁- bis C₄)-alkylgruppe, eine verzweigte oder unverzweigte C₁- bis C₄-Aminoalkylgruppe, eine verzweigte oder unverzweigte C₁- bis C₄Alkykamino-(C₁- bis C₄)-alkylgruppe oder eine verzweigte oder unverzweigte C₁- bis C₄-Dialkykamino-(C₁- bis C₄)-alkylgruppe.

Im Rahmen dieser Ausführungsform kann es besonders bevorzugt sein, wenn bei dem m-Phenylendiaminderivat der Formel (I) mindestens einer der Substituenten R₁, R₂, R₃ und R₄ für eine verzweigte oder unverzweigte C₁- bis C₄-Alkylgruppe oder eine verzweigte oder unverzweigte C₁- bis C₄-Monohydroxyalkylgruppe stehen. Ganz besonders bevorzugt sind m-Phenylendiaminderivate der Formel (I), bei denen mindestens einer der Substituenten R₁, R₂, R₃ und R₄ für eine Hydroxyethylgruppe steht.

Weiterhin kann es erfindungsgemäß bevorzugt sein, wenn mindestens zwei der Substituenten R₁, R₂, R₃ und R₄ für Wasserstoff stehen.

Ferner können m-Phenylendiaminderivat der Formel (I) bevorzugt sein, bei denen der Substituent R₁ dieselbe Bedeutung hat wie der Substituent R₃.

Im Rahmen dieser Ausführungsform kann es weiterhin bevorzugt sein, wenn zusätzlich der Substituent R₂ dieselbe Bedeutung hat wie der Substituent R₄.

Weiterhin kann es erfindungsgemäß bevorzugt sein, dass der Substituent R₅ für Wasserstoff steht.

Ferner hat es sich erfindungsgemäß bevorzugt erwiesen, wenn X für ein Chloratom, ein Bromatom oder ein Fluoratom steht, die m-Phenylendiaminderivate der Formel (I), bei denen X für ein Chloratom haben sich als ganz besonders bevorzugt erwiesen.

Als erfindungsgemäß ganz besonders bevorzugter Vertreter der m-Phenylendiaminderivate der Formel (I) sei explizit 2-({3-Chlor-5-[(2-hydroxyethyl)amino]phenyl}amino)ethanol genannt.

Neben den m-Phenylendiaminderivaten der Formel (I) können die erfindungsgemäßen Färbemittel ferner mindestens eine Entwicklerkomponente enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Amino-pyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen.

Besonders bevorzugte p-Phenylendiamine sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Bevorzugte zweikernige Entwicklerkomponenten sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-pherlylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten sind N,N'-Bis(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen.

Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind Insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-6,6-diaminopyrimidin und 2,5,6-Triaminopyrimidin,

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7 -Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als erfindungsgemäß besonders geeignet haben sich die folgenden Kuppler/Entwicklerkombinationen erwiesen:
- 2-({3-Chlor-5-[(2-hydroxyethyl)amino]phenyl}amino)ethanol / p-Toluylendiamin,
- 2-({3-Chlor-5-[(2-hydroxyethyl)amino]phenyl}amino)ethanol /1-(2-Hydroxyethyl)-2,6-diaminobenzol,
- 2-({3-Chlor-5-[(2-hydroxyethyl)amino]phenyl}amino)ethanol / Bis-(2-hydroxy-5-aminophenyl)-methan,
- 2-({3-Chlor-5-[(2-hydroxyethyl)amino]phenyl}amino)ethanol / 2,4,5,6-Tetraaminopyrimidin und
- 2-({3-Chlor-5-[(2-hydroxyethyl)amino]phenyl}amino)ethanol / N,N-Bis-(2-hydroxyethyl)-p-phenylendiamin

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung können die Färbemittel mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins;

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den erfindungsgemäßen m-Phenylendiaminderivaten der Formel (I) können die erfindungsgemäßen Färbemittel in einer weiteren bevorzugten Ausführungsform der vorliegenden Erfindung zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-Imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoesslgsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise VinylpyrrolidonNinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol, Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamells, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1999, verwiesen.

Die erfindungsgemäßen Mittel enthalten die Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol beziehungsweise Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Erfindung ist ein Verfahren zur Färbung keratinischer Fasern, bei dem ein erfindungsgemäßes Haarfärbemittel auf die Fasern aufgetragen wird und nach einer Einwirkzeit wieder abgespült wird.

Ein dritter Gegenstand der vorliegenden Erfindung ist nämlich2-({3-Chlor-5-[(2-hydroxyethyl)amino]phenyl}amino)ethanol.

Ein vierter Gegenstand der vorliegenden Erfindung sind die erste und die zweite Zwischenstufen der Synthese des erfindungsgemäß ganz besonders bevorzugten m-Phenylendiamins, nämlich 3-[3-Chlor-5-(2-oxo-1,3-oxazolidin-3-yl)phenyl]-1,3-oxazolidin-2-on und 2-Chlorethyl 3-chlor-5-{[(2-chlorethoxy)carbonyl]amino}phenylcarbamat.

### Ausführungsbeispiele

### 1 Synthesen

### 1.1 2-({3-Chlor-5-[(2-hydroxyethyl)amino]phenyl}amino)ethanol

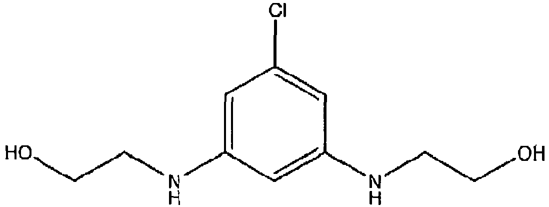

1.1.1 2-Chlorethyl 3-chlor-5-{[(2-chlorethoxy)carbonyl]amino}phenylcarbamat 25 g 5-Chlor-m-phenylendiamin und 58 g Calciumcarbonat wurden in 0,5 l Dioxan vorgelegt und auf 90 °C erwärmt. Innerhalb von 15 min wurden 67 g 2-Chlorethylchloroformiat zugegeben und es wurde weitere 4 h bei dieser Temperatur gerührt. Im Anschluss ließ man den Ansatz abkühlen und filtrierte die Mineralsalze ab. Das Filtrat wurde auf Eiswasser gegossen, das Produkt abgesaugt und im Vakuum getrocknet.

| | |
|---|---|
| Ausbeute: | 62 g (97 %) |
| Schmelzpunkt: | 83 - 90 °C |

1.1.2 4.2 3-[3-Chlor-5-(2-oxo-1,3-oxazolidin-3-yl)phenyl]-1,3-oxazolidin-2-on 200 ml Natronlauge (20 %ig) wurden vorgelegt und auf 45 °C erwärmt. Im Anschluss wurden 62 g 2-Chlorethyl 3-chlor-5-{[(2-chlorethoxy)carbonyl]amino}phenylcarbamat gelöst in 200 ml Dioxan hinzugegeben, und der Ansatz wurde weitere 2 h bei Raumtemperatur nachgerührt. Anschließend wurde der Ansatz auf Eis gegossen und mit Salzsäure neutralisiert. Das ausgefallene Produkt wurde abgesaugt und über Nacht im Vakuum getrocknet.

| | |
|---|---|
| Ausbeute: | 46 g (96 %) |
| Schmelzpunkt: | 195 - 196 °C |

### 1.1.3 4.3 2-({3-Chlor-5-[(2-hydroxyethyl)amino]phenyl}amino)ethanol (1)

300 ml 20 %ige ethanolische KOH und 46 g 3-[3-Chlor-5-(2-oxo-1,3-oxazolidin-3-yl)phenyl]-1,3-oxazolidin-2-on wurden vorgelegt und 3 h unter Rückfluss erwärmt. Danach wurde mit HCl neutralisiert und mehrfach mit Essigester extrahiert. Die vereinigten organischen Phasen wurden am Rotavapor bis zur Trockne eingeengt, wobei eine braune viskose Substanz erhalten wurde, die im Anschluss im Kugelrohr im Vakuum destilliert wurde (250 - 300 °C, Hochvakuum).

| | |
|---|---|
| Ausbeute: | 13 g (35 %) |

### 2 Ausfärbungen

### 2.1 Versuchsdurchtührung

Für die Herstellung der Färbecreme wurden 50g einer Cremebasis in einem 250ml Becherglas eingewogen und bei 80°C geschmolzen. Die verwendete Cremebasis hatte die folgende Zusammensetzung:

| | |
|---|---|
| Hydrenol^{®} D¹ | 17,0 Gew.-% |
| Lorol^{®} tech.² | 4,0 Gew.-% |
| Texapon^{®} NSO³ | 40,0 Gew.-% |
| Dehyton^{®} K⁴ | 25,0 Gew.-% |
| Eumulgin^{®} B2⁵ | 1,5 Gew.-% |
| Wasser | 12,5 Gew.-% |

| | |
|---|---|
| ¹ C₁₆₋₁₈-Fettalkohol (INCI-Bezeichnung: Cetearyl alcohol) (Cognis) ² C₁₂₋₁₈-Fettalkohol (INCI-Bezeichnung: Coconut alcohol) (Cognis) ³ Laurylethersulfat, Natriumsalz (ca. 27,5% Aktivsubstanz; INCI-Bezeichnung: Sodium Laureth Sulfate) (Cognis) ⁴ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (Cognis) ⁵ Cetylstearylalkohol mit ca. 20 EO-Einheiten (INCI-Bezeichnung: Ceteareth-20) (Cognis) | |

Es wurden jeweils 1/400 Mol der Entwickler- bzw. Kupplerkomponente getrennt in destilliertem Wasser suspendiert bzw. unter Erwärmen gelöst. Anschließend wurde Ammoniak (<1 ml; 25%ige Ammoniaklösung) zugegeben bis der pH-Wert zwischen 9 und 10 lag.

Die gelösten Farbstoffvorprodukte wurden nacheinander in die heiße Creme eingearbeitet. Anschließend wurde mit destilliertem Wasser auf 97g aufgefüllt und mit Ammoniak ein pH-Wert von 9,5 eingestellt. Nach Auffüllen mit destilliertem Wasser auf 100 g wurde der Ansatz kaltgerührt (< 30°C), wobei eine homogene Creme entstand. Für die Ausfärbungen wurden jeweils 25 g Färbecreme mit 25 g der folgenden Oxidationsmittelzubereitung vermischt.

| | |
|---|---|
| Dipicolinsäure | 0,1 Gew.-% |
| Natriumpyrophosphat | 0,03 Gew.-% |
| Turpinal^{®} SL⁶ | 1,50 Gew.-% |
| Texapon^{®} N28⁷ | 2,00 Gew.-% |
| Acrysol^{®} 22⁸ | 0,60 Gew.-% |
| Wasserstoffperoxid, 50 %ig | 12,00 Gew.-% |
| Natronlauge, 45%ig | 0,80 Gew.-% |
| Wasser | ad 100 Gew.-% |

| | |
|---|---|
| ⁶ 1-Hydroxyethan-1,1-diphosphonsäure (ca. 58-61% Aktivsubstanzgehalt; INCI-Bezeichnung: Etidronic Acid, Aqua (Water)) (Solutia) ⁷ Laurylethersulfat-Natrium-Salz (mind. 26,5 % Aktivsubstanzgehalt; INCI-Bezeichnung: Sodium Laureth Sulfate) (Cognis) ⁹ Acrylpolymer (ca. 29.5 - 30.5% Festkörper in Wasser; INCI-Bezeichnung: Acrylates/Steareth-20 Methacrylate Copolyme) | |

In jede der so erhaltenen Mischungen wurde eine Haarsträhne (80 % ergraut; 330 mg bis 370 mg schwer) gegeben. Anschließend wurden die Mischungen und die Haarsträhnen auf jeweils ein Uhrglas gegeben und die Haarsträhnen in die Färbecremes gut eingebettet. Nach 30 Minuten (±1 Minute) Einwirkzeit bei Raumtemperatur wurden die Haarsträhnen entnommen und mit einer wässrigen Texapon^{®} EVR-Lösung⁹ so oft gewaschen, bis der Farbüberschuß entfernt war. Die Haarsträhnen wurden an der Luft getrocknet und ihr Farbton unter der Tageslichtlampe (Farbprüfgerät HE240A) bestimmt und notiert (Taschenlexikon der Farben, A. Kornerup u. J.H. Wanscher, 3. unveränderte Auflage 1981, MUSTER-SCHMIDT Verlag; Zürich, Göttingen).
⁹ Laurylethersulfat-Natrium-Salz mit speziellen Zusätzen (ca. 34 bis 37% Aktivsubstanzgehalt; INCI-Bezeichnung: Sodium Lauryl Sulfate, Sodium Laureth Sulfate, Lauramide MIPA, Cocamide MEA, Glycol Stearate, Laureth-10) (Cognis)

Die bei den Ausfärbungs-Untersuchungen erhaltenen Ergebnisse sind in den nachstehenden Tabellen aufgeführt.

### 2.2 Ausfärbung mit 2-({3-Chlor-5-[(2-hydroxyethyl)amino]phenyl}amino)ethanol

| Entwicklerkomponente | Ausfärbung |
|---|---|
| p-Toluylendiamin-Sulfat | graurubin |
| 2,4,5,6-Tetraaminopyrimidin-Sulfat | fuchsrot |
| p-Aminophenol | rotbraun |
| 4,5-Diamino-1-(2-hydroxyethyl)pyrazol-Sulfat | dunkelviolett |
| 2-(2,5-Diaminophenyl)ethanol-Sulfat | rubin |
| 4-Amino-3-methylphenol | braunorange |
| 4-Amino-2-[(5-amino-2-hydroxyphenyl)methyl]phenol-Dihydrochlorid | braunrot |
| N,N-Bis-(2-hydroxyethyl)-p-phenylendiamin-Sulfat | tiefviolett |

## Patentansprüche

1. Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 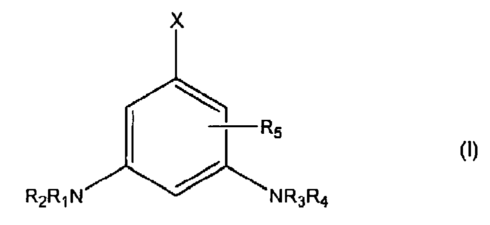 wobei
R₁, R₂, R₃ und R₄ unabhängig voneinander für ein Wasserstoffatom, eine verzweigte oder unverzweigte C₁- bis C₄-Alkylgruppe, eine verzweigte oder unverzweigte C₁- bis C₄-Monohydroxyalkylgruppe, eine verzweigte oder unverzweigte C₂- bis C₄-Polyhydroxyalkylgruppe, eine verzweigte oder unverzweigte C₁- bis C₄-Alkoxy-(C₁- bis C₄)-alkylgruppe, eine verzweigte oder unverzweigte C₁- bis C₄-Aminoalkylgruppe, eine verzweigte oder unverzweigte C₁- bis C₄-Alkykamino-(C₁- bis C₄)-alkylgruppe oder eine verzweigte oder unverzweigte C₁- bis C₄-Dialkykamino-(C₁- bis C₄)-alkylgruppe stehen.
R₅ für ein Wasserstoffatom, eine C₁- bis C₄-Alkylgruppe, eine C₁- bis C₄-Monohydroxyalkylgruppe, eine C₂- bis C₄- Polyhydroxyalkylgruppe, eine C₁- bis C₄-Alkoxy-(C₁- bis C₄)-alkylgruppe oder ein Halogenatom steht, und
X für ein Halogenatom steht.
mit der Maßgabe, dass
- mindestens einer der Substituenten R₁, R₂, R₃ und R₄ für Wasserstoff steht, und
- mindestens einer der Substituenten R₁, R₂, R₃ und R₄ von Wasserstoff verschieden ist.

2. Mittel nach Anspruch 1, **dadurch gekennzeichnet, dass** es ein m-Phenylendiaminderivat der Formel (I) enthält, bei dem mindestens einer der Substituenten R₁, R₂, R₃ und R₄ für eine verzweigte oder unverzweigte C₁- bis C₄-Alkylgruppe oder eine verzweigte oder unverzweigte C₁- bis C₄-Monohydroxyalkylgruppe steht.

3. Mittel nach Anspruch 2, **dadurch gekennzeichnet, dass** es ein m-Phenylendiaminderivat der Formel (I) enthalt, bei dem mindestens einer der Substituenten R₁, R₂, R₃ und R₄ für eine Hydroxyethylgruppe steht.

4. Mittel nach einem der Ansprüche 1 bis 3, **dadurch gekennzeichnet, dass** es ein m-Phenylendiaminderivat der Formel (I) enthält, bei dem mindestens zwei der Substituenten R₁, R₂, R₃ und R₄ für Wasserstoff stehen.

5. Mittel nach einem der Ansprüche 1 bis 4, **dadurch gekennzeichnet, dass** der Substituent R₁ dieselbe Bedeutung hat wie der Substituent R₃.

6. Mittel nach Anspruch 5, **dadurch gekennzeichnet, dass** der Substituent R₂ dieselbe Bedeutung hat wie der Substituent R₄.

7. Mittel nach einem der Ansprüche 1 bis 6, **dadurch gekennzeichnet, dass** der Substituent R₅ für Wasserstoff steht.

8. Mittel nach einem der Ansprüche 1 bis 7, **dadurch gekennzeichnet, dass** X für ein Chloratom, ein Bromatom oder ein Fluoratom, vorzugsweise für ein Chloratom steht.

9. Mittel nach einem der Ansprüche 1 bis 8, **dadurch gekennzeichnet, dass** es als m-Phenylendiaminderivat 2-({3-Chlor-5-[(2-hydroxyethyl)amino]phenyl}amino)ethanol enthält.

10. Mittel nach einem der Ansprüche 1 bis 9, **dadurch gekennzeichnet, dass** es mindestens eine Entwicklerkomponente enthält.

11. Mittel nach einem der Ansprüche 1 bis 10, **dadurch gekennzeichnet, dass** es mindestens eine weitere Kupplerkomponente enthält.

12. Mittel nach einem der Ansprüche 1 bis 11, **dadurch gekennzeichnet, dass** es weiterhin mindestens einen direktziehenden Farbstoff enthält.

13. Mittel nach Anspruch 12, **dadurch gekennzeichnet, dass** der direktziehende Farbstoff kationisch ist.

14. Verfahren zur Färbung keratinischer Fasern, bei dem ein Mittel nach einem der Ansprüche 1 bis 13 auf die Fasern aufgetragen wird und nach einer Einwirkzeit wieder abgespült wird.

15. 2-({3-Chlor-5-[(2-hydroxyethyl)amino]phenyl}amino)ethanol

16. 3-[3-Chlor-5-(2-oxo-1,3-oxazolidin-3-yl)phenyl]-1,3-oxazolidin-2-on

17. 2-Chlorethyl 3-chlor-5-{[(2-chlorethoxy)carbonyl]amino}phenylcarbamat

## Claims

1. Agent for dyeing keratinic fibers, in particular human hair, containing in a cosmetically acceptable
vehicle, as the coupler component, at least one m-phenylenediamine derivative of formula (I) 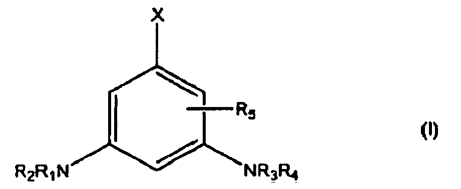 where
R₁, R₂, R₃ and R₄, independently of one another, stand for a hydrogen atom, a branched or unbranched C₁ to C₄ alkyl group, a branched or unbranched C₁ to C₄ monohydroxyalkyl group, a branched or unbranched C₂ to C₄ polyhydroxyalkyl group, a branched or unbranched C₁ to C₄ alkoxy-(C₁ to C₄)-alkyl group, a branched or unbranched C₁ to C₄ aminoalkyl group, a branched or unbranched C₁ to C₄ alkylamino-(C₁ to C₄)-alkyl group or a branched or unbranched C₁ to C₄ dialkylamino-(C₁ to C₄)-alkyl group,
R₅ stands for a hydrogen atom, a C₁ to C₄ alkyl group, a C₁ to C₄ monohydroxyalkyl group, a C₂ to C₄ polyhydroxyalkyl group, a C₁ to C₄ alkoxy-(C₁ to C₄)-alkyl group or a halogen atom, and
X stands for a halogen atom,
with the provision that
- at least one of the substituents R₁, R₂, R₃ and R₄ stands for hydrogen, and
- at least one of the substituents R₁, R₂, R₃ and R₄ is different from hydrogen.

2. Agent according to claim 1, **characterized in that** it contains an m-phenylenediamine derivative of formula (I), wherein at least one of the substituents R₁, R₂, R₃ and R₄ stands for a branched or unbranched C₁ to C₄ alkyl group or a branched or unbranched C₁ to C₄ monohydroxyalkyl group.

3. Agent according to claim 2, **characterized in that** it contains an m-phenylenediamine derivative of formula (I), wherein at least one of the substituents R₁, R₂, R₃ and R₄ stands for a hydroxyethyl group.

4. Agent according to any one of claims 1 to 3, **characterized in that** it contains an m-phenylenediamine derivative of formula (I), wherein at least two of the substituents R₁, R₂, R₃ and R₄ stand for hydrogen.

5. Agent according to any one of claims 1 to 4, **characterized in that** the substituent R₁ has the same meanings as the substituent R₃.

6. Agent according to claim 5, **characterized in that** the substituent R₂ has the same meanings as the substituent R₄.

7. Agent according to any one of claims 1 to 6, **characterized in that** the substituent R₅ stands for hydrogen.

8. Agent according to any one of claims 1 to 7, **characterized in that** X stands for a chlorine atom, a bromine atom or a fluorine atom, preferably a chlorine atom.

9. Agent according to any one of claims 1 to 8, **characterized in that** it contains, as the m-phenylenediamine derivative, 2-({3-chloro-5-[(2-hydroxyethyl)amino]phenyl}amino)ethanol.

10. Agent according to any one of claims 1 to 9, **characterized in that** it contains at least one developer component.

11. Agent according to any one of claims 1 to 10, **characterized in that** it contains at least one additional coupler component.

12. Agent according to any one of claims 1 to 11, **characterized in that** it also contains at least one direct colorant.

13. Agent according to claim 12, **characterized in that** the direct colorant is cationic.

14. Method for dyeing keratinic fibers, wherein an agent according to any one of claims 1 to 13 is applied to the fibers and is rinsed out again after a treatment time.

15. 2-({3-Chloro-5-[(2-hydroxyethyl)amino]phenyl}amino)ethanol.

16. 3-[3-Chloro-5-(2-oxo-1,3-oxazolidin-3-yl)pheny]-1,3-oxazoidin-2-one.

17. 2-Chlorothyl-3-chloro-5-{[(2-chloroethoxy)carbonyl]amino}phenylcarbamate.

## Revendications

1. Agent de coloration de fibres de kératine, en particulier des cheveux humains, contenant dans un support cosmétiquement acceptable, comme composant coupleur au moins un dérivé m-phénylènediamine la de formule (I) 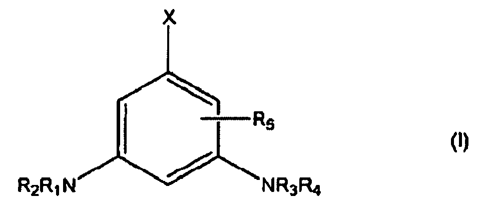 où
R₁, R₂, R₃ et R₄ représentent indépendamment un atome d'hydrogène, un groupe alkyle en C₁ à C₄ ramifié ou non ramifié, un groupe monohydroxyalkyle en C₁ à C₄ ramifié ou non ramifié, un groupe polyhydroxyalkyle en C₂ à C₄ ramifié ou non ramifié, un groupe alcoxy en C₁ à C₄-alkyle en C₁ à C₄, un groupe aminoalkyle en C₁ à C₄ ramifié ou non ramifié, un groupe alkykamino en C₁ à C₄-alkyle en C₁ à C₄ ramifié ou non ramifié ou un groupe dialkykamino en C₁ à C₄-alkyle en C₁ à C₄ ramifié ou non ramifié,
R₅ représente un atome d'hydrogène, un groupe alkyle en C₁ à C₄, un groupe monohydroxyalkyle en C₁ à C₄, un groupe polyhydroxyalkyle en C₂ à C₄, un groupe alcoxy en C₁ à C₄-alkyle en C₁ à C₄ ou un atome d'halogène, et
X représente un atome d'halogène,
avec les conditions que
- l'un au moins des substituants R₁, R₂, R₃ et R₄ soit un atome d'hydrogène, et
- l'un au moins des substituants R₁, R₂, R₃ et R₄ soit différent de l'hydrogène.

2. Agent selon la revendication 1, **caractérisé en ce qu'**il comprend un dérivé m-phénylènediamine de la formule (I), l'un au moins des substituants R₁, R₂, R₃ et R₄ représentant un groupe alkyle en C₁ à C₄ ramifié ou non ramifié ou un groupe monohydroxyalkyle en C₁ à C₄ ramifié ou non ramifié.

3. Agent selon la revendication 2, **caractérisé en ce qu'**il contient un dérivé m-phénylènediamine de la formule (I), l'un au moins des substituants R₁, R₂, R₃ et R₄ représentant un groupe hydroxyéthyle.

4. Agent selon l'une des revendications 1 à 3, **caractérisé en ce qu'**il contient un dérivé m-phénylènediamine de la formule (I), deux au moins des substituants R₁, R₂, R₃ et R₄ représentant un atome d'hydrogène.

5. Agent selon l'une des revendications 1 à 4, **caractérisé en ce que** le substituant R₁ a la même signification que le substituant R₃.

6. Agent selon la revendication 5, **caractérisé en ce que** le substituant R₂ a la même signification que le substituant R₄.

7. Agent selon l'une des revendications 1 à 6, **caractérisé en ce que** le substituant R₅ est un atome d'hydrogène.

8. Agent selon l'une des revendications 1 à 7, **caractérisé en ce que** X représente un atome de chlore, un atome de brome ou un atome de fluor, de préférence un atome de chlore.

9. Agent selon l'une des revendications 1 à 8, **caractérisé en ce que** le dérivé m-phénylènediamine qu'il contient est le 2-({3-[chloro-5-(2-hydroxyéthyl)amino]phényl}amino)éthanol.

10. Agent selon l'une des revendications 1 à 9, **caractérisé en ce qu'**il contient au moins une base d'oxydation.

11. Agent selon l'une des revendications 1 à 10, **caractérisé en ce qu'**il contient au moins un autre coupleur.

12. Agent selon l'une des revendications 1 à 11, **caractérisé en ce qu'**il contient en outre au moins un colorant montant directement sur les fibres.

13. Agent selon la revendication 12, **caractérisé en ce que** le colorant montant directement sur les fibres est cationique.

14. Procédé de coloration de fibres de kératine dans lequel un agent selon l'une des revendications 1 à 13 est appliqué sur les fibres et est rincé à nouveau au bout d'un temps d'action.

15. 2-({3-chloro-5 -[(2-hydroxyéthyl)amino]phényl}amino)éthanol.

16. 3-[3-chloro-5-(2-oxo-1,3-oxazolidine-3-yl)phényl]-1,3-oxazolidine-2-one.

17. 3-chloro-5-{[(2-chloroéthoxy)carbonyl]amino}phénylcarbamate de 2-chloroéthyle.
